(19) Europäisches Patentamt
European Patent Office
Office européen des brevets

(11) Publication number: **0 010 787**
**B1**

# (12) EUROPEAN PATENT SPECIFICATION

(45) Date of publication of patent specification: **12.05.82**

(51) Int. Cl.³: **C 07 D 209/52**

(21) Application number: **79200518.3**

(22) Date of filing: **17.09.79**

## (54) Isomerisation of 3-azabicyclo(3.1.0)hexane derivatives.

(30) Priority: **27.09.78 GB 3833078**

(43) Date of publication of application:
**14.05.80 Bulletin 80/10**

(45) Publication of the grant of the patent:
**12.05.82 Bulletin 82/19**

(84) Designated Contracting States:
**AT BE CH DE FR GB IT NL SE**

(56) References cited:
**FR - A - 2 324 236**

(73) Proprietor: **SHELL INTERNATIONALE RESEARCH MAATSCHAPPIJ B.V.**
**Carel van Bylandtlaan 30**
**NL-2596 HR DEN HAAG (NL)**

(72) Inventor: **Searle, Robert John Griffith**
**Highworth Stockers Hill Rodmersham Green**
**Sittingbourne Kent (GB)**
Inventor: **Day, Janet Anne**
**3, Palmerstone Walk**
**Sittingbourne Kent (GB)**
Inventor: **Wood, Derek Alexander**
**76 Sterling Road**
**Sittingbourne Kent (GB)**
Inventor: **Mason, Ronald Frank**
**"Kingswood" Westwell**
**Near Ashford Kent (GB)**

(74) Representative: **Rogers, Roy Charles et al,**
**4 York Road**
**London SE1 7NA (GB)**

**EP 0 010 787 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

# Isomerisation of 3-azabicyclo[3.1.0]hexane derivatives

The present invention relates to a process for the isomerisation of a cis or trans 2-cyano-3-azabicyclo[3.1.0]hexane into the corresponding trans or cis isomer.

2-Cyano-3-azabicyclo[3.1.0]hexanes can conveniently be used as starting materials for the preparation of the corresponding 2-carboxy-3-azabicyclo[3.1.0]hexanes or derivatives thereof, which exhibit interesting biological properties, especially with respect to the sterilisation of male anthers in plants, in particular in small grain cereals. In this connection reference is made to French Patent Specification 2,324,236.

It will be appreciated that 2-cyano-3-azabicyclo[3.1.0]hexanes exhibit both optical and geometric isomerism. Geometric isomerism with respect to the cyano group is of the cis/trans nature, reflecting the mutual positions of the 2-cyano group and the 3,4-methano bridging group. As it is possible that biologically active isomers will exhibit different degrees of activity, there is a need to convert the less active isomer into the more active isomer, to convert the precursor of a less active isomer into the precursor of the more active isomer or directly into the more active isomer itself. There is also a need for a suitable isomerisation process in the event that the cis and trans isomers are produced or available as mixtures containing predominantly the less desired isomer.

The invention therefore provides a process for the isomerisation of a compound of the general formula

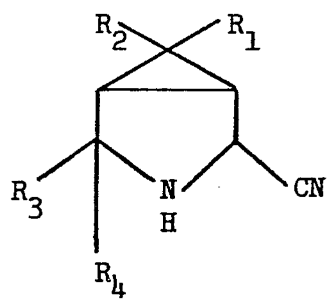

(I)

wherein the group CN and the

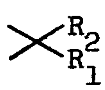

moiety are in a cis or trans configuration and $R_1$, $R_2$, $R_3$ and $R_4$ each independently represents a hydrogen or halogen atom or an alkyl group with up to 6 carbon atoms which may optionally be substituted by up to 3 halogen atoms, into the corresponding trans or cis isomer by contacting the compound according to formula I with a solvent in the presence of a compound containing, or capable under the isomerisation conditions of generating, free cyanide moieties.

It is an advantage of the present process that the isomerisation can be carried out under mild reaction conditions, which minimise condensation, polymerisation and/or decomposition reactions of the nitrile concerned.

Under suitable conditions, the isomerisation may proceed close to the thermodynamically controlled equilibrium of the isomers concerned. Separation of the two isomers, e.g. in the case of cis and trans 2-cyano-3-azabicyclo[3.1.0]hexane ($R_1$, $R_2$, $R_3$ and $R_4$ each being hydrogen) by treating the product mixture with benzene sulphonic acid or a toluene sulphonic acid to form the corresponding sulphonic acid salts which exhibit very different solubility characteristics as described and claimed in co-pending application 792005191 may be effected if desired, after which the unwanted isomer can be subjected again to the isomerisation process according to the present invention. This isomerisation/separation process may be repeated several times, in order to provide the maximum amount of the desired isomer. Mixtures which contain an "enriched" amount of a particular isomer, e.g. as obtained by certain working-up procedures such as preferential crystallisation, can be subjected to the isomerisation process according to the present invention.

The process according to the invention is carried out in the presence of a compound containing, or capable of generating under the isomerisation conditions, free cyanide moieties. "Free cyanide moieties" should be understood to include discrete CN moieties, whether charged (wholly or partially ionic) or not, and whether present permanently or only transiently in the reaction medium. Suitable cyanides include cyanide salts, for example an alkali or earth alkaline metal cyanide such as sodium, potassium or barium cyanide, ammonium cyanide or an alkyl-substituted ammonium cyanide which may contain up to 4 alkyl groups each of which may contain up to 4 carbon atoms. Hydrogen cyanide can also be used as the cyanide in the process according to the present invention, as can cyanide containing exchange resins. Compounds which are capable of generating hydrogen cyanide can be

used, such as aldehyde and ketone cyanohydrins, for instance acetone cyanohydrin, methyl ethyl ketone cyanohydrin and acetaldehyde cyanohydrin. More than one cyanide may be present during the isomerisation process.

The amount of free cyanide present in the reaction mixture is not critical and can vary between wide limits. Amounts of added cyanide compound of up to 200 molar % calculated on starting material can be used conveniently. Good results are normally obtained by adding a cyanide in amounts in the range of from 1% to 100% m, amounts in the range of 5% m up to 50% m being preferred.

Suitable solvents to be used in the process according to the present invention are alkanols with up to 6 carbon atoms, such as methanol, ethanol, propanol, isopropanol, butanol or pentanol. The alcohols may contain a substantial amount of water, e.g. amounts up to 25% by weight, which may facilitate the incorporation of added cyanide in the reaction medium. Ketones can also be used as the solvent; suitable ketones includes dimethyl ketone, methyl ethyl ketone, diethyl ketone, methyl propyl ketone and methyl isobutyl ketone. Aromatic hydrocarbons such as benzene, toluene and the xylenes, and ethers such as diethylether, monocyclic ethers such as tetrahydrofuran and dioxan and macrocyclic polyethers commonly referred to as "crown ethers", such as 15-crown-5 and 18-crown-6, can be used conveniently. A further class of solvents comprises sulphones and sulphoxides, such as sulpholane and dimethylsulphoxide.

Preference is given to the use of lower alkanols, in particular isopropanol. Mixtures of solvents may be advantageous. The use of macrocyclic polyethers may be advantageous as they possess a high complexing power for alkali and earth alkaline metal ions thus rendering the cyanide ion more active, if a cyanide salt of one of these metals is used.

Carboxylic acids such as acetic acid and propanoic acid can be used as solvents provided that care is taken to prevent any substantial hydrolysis of the cyano group in the compound of formula I.

The process according to the present invention can be carried out at moderate temperatures, e.g. from ambient temperature up to 200°C. Temperatures below 120°C, in the range of 80—100°C, especially around 90°C are preferred.

The isomerisation process according to the present invention is preferably carried out by stirring the reaction mixture under moderate heating for about two hours. After removal of the solvent, for example by evaporation, the residue may be extracted with an organic solvent in which added cyanide is not, or only sparingly, soluble, the extract dried and the product obtained by removal of the organic solvent.

It may be advantageous to convert the product comprising a cis and trans 2-cyano-3-azabicyclo 3.1.0 hexane according to formula I into the corresponding carboxylic acid and/or esters rather than separating the isomers obtained. To this extent the reaction mixture can be treated either as such or after removal of added cyanide as described hereinabove, with a strong acid such as hydrochloric acid which will normally give the acids almost quantitatively. If desired, the acids can be converted into derivatives such as the corresponding alkyl esters by methods known per se.

The invention is illustrated by means of the following Examples.

Example 1

Pure trans 2-cyano-3-azabicyclo[3.1.0]hexane (20 g) was dissolved in isopropanol (80 ml) containing sodium cyanide (5 g). The mixture was stirred and refluxed for 3 hours. The isopropanol was then removed by evaporation and the residue dissolved in diethyl ether (50 ml), and filtered to remove any cyanide residues. Distillation gave 2-cyano-3-azabicyclo[3.1.0]hexane (19.0 g) containing 44% cis isomer.

The experiment was repeated whilst allowing a reflux time of 1½ hours. The final mixture contained 41% of cis isomer. The 2-cyano-3-azabicyclo[3.1.0]hexane isomers were characterised as described in co-pending application 4,107.

Example 2

A mixture of trans and cis 2-cyano-3-azabicyclo[3.1.0]hexane (0.4 g; trans/cis ratio 92/8) and sodium cyanide (0.1 g) in isopropanol (1.6 ml) was stirred under reflux for 1½ hours. The mixture was then cooled in ice and boiled down. The residue was extracted with diethyl ether, dried over anhydrous sodium sulphate and the ether was evaporated. The amount of product recovered was 0.31 g and $^{13}C$ NMR analysis indicated the presence of 35—38% of cis 2-cyano-3-azabicyclo[3.1.0]hexane.

Example 3

A mixture of trans and cis 2-cyano-3-azabicyclo[3.1.0]hexane (0.4 g; trans/cis ratio 92/8) and sodium cyanide (0.1 g) in dry absolute ethanol (1.6 ml) was refluxed under stirring for 1½ hours. The mixture was then boiled down and the residue partially dissolved in diethyl ether. The ether solution was dried over anhydrous sodium sulphate, filtered and the ether was evaporated. The amount of product recovered was 0.3 g and $^{13}C$ NMR analysis indicated the presence of 28% of cis 2-cyano-3-azabicyclo[3.1.0]hexane.

The experiment was repeated using n-pentanol (1.6 g) as the solvent. After evaporation at 80°C/1 mm, the residue was partially dissolved in diethyl ether. After filtration, drying over anhydrous

sodium sulphate and evaporation of the ether 0.22 g of product was obtained which contained 33% of cis-isomer ($^{13}C$ NMR analysis).

### Example 4

A mixture of trans and cis 2-cyano-3-azabicyclo[3.1.0]hexane (0.4 g; trans/cis ratio 92/8) and potassium cyanide (0.13 g) in toluene (1.6 ml) containing a trace of 18-crown-6 was refluxed under stirring for 1 hour and subsequently left to cool overnight. The mixture was then poured into water (75 ml) shaken with an equal volume of toluene followed by boiling down the separated toluene layer. The amount of product recovered was 0.2 g and $^{13}C$ NMR analysis indicated the presence of 33—34% of cis 2-cyano-3-azabicyclo[3.1.0]hexane.

Isomerisation was also obtained by stirring a starting material as described hereinabove for 7 days at room temperature.

### Example 5

A spatula full of exchange resin Amberlite 410 (CN) (AMBERLITE is a Trade Mark) was added to a solution of trans 2-cyano-3-azabicyclo[3.1.0]hexane (0.5 g) in isopropanol (2.5 ml). The suspension was stirred and heated under reflux for 1 hour. The resin was filtered off and the isopropanol evaporated. The amount of product recovered was 0.5 g (yellow liquid) and $^{13}C$ NMR analysis indicated the presence of 32% cis 2-cyano-3-azabicyclo[3.1.0]hexane.

### Example 6

A solution of trans and cis 2-cyano-3-azabicyclo[3.1.0]hexane (0.4 g; trans/cis ratio 92/8) and sodium cyanide (0.1 g) in dry dimethylsulphoxide (1.6 ml) was stirred in a flask and heated at 200°C for 5 minutes under a nitrogen atmosphere. Then the flask was cooled in an ice bath and then the solution which had turned brown was subjected to high vacuum distillation to remove dimethyl sulphoxide. The residue was partly dissolved in diethyl ether; the etheral layer was then dried over anhydrous sodium sulphate and evaporated down. The amount of product obtained was 0.35 g (pale brown liquid) and $^{13}C$ NMR analysis indicated the presence of about 30% of cis 2-cyano-3-azabicyclo[3.1.0]hexane.

### Example 7

A solution of trans 2-cyano-3-azabicyclo[3.1.0]hexane (0.5 g) and sodium cyanide (0.23 g) in glacial acetic acid (30 ml) was stirred and heated under reflux for two hours under a nitrogen atmosphere. The yellow solution was then boiled down and the solid residue extracted with diethyl ether (3 × 100 ml). The ethereal layers were dried over anhydrous sodium sulphate and evaporated down. The resulting oil was then dissolved in 5 N HCl (50 ml) and heated under reflux for 6 hours. The solution was evaporated down and then applied to an Amberlite 1R 120 ($H^+$) column, the eluent being $NH_4OH$. The amount of product obtained was 0.6 g and from NMR analysis it appeared to be a mixture of trans and cis 2-carboxy-3-azabicyclo[3.1.0]hexane containing 20—25% of the cis acid, the remainder being the trans acid.

A further experiment was carried out by refluxing under nitrogen for two days a solution of trans 2-cyano-3-azabicyclo[3.1.0]hexane (0.5 g) and a large excess of sodium cyanide (2.01 g) in acetic acid (50 ml) in the presence of a small amount of concentrated hydrochloric acid (0.2 ml). The mixture was then poured onto ice, basified by addition of ammonia (60 ml) and extracted with chloroform. The chloroform layer was then dried over anhydrous sodium sulphate and evaporated down. The resulting dark brown oil was dissolved in 5 N HCl (50 ml) and heated under reflux for 6 hours. The solution was evaporated down and applied to an Amberlite 1R 120 ($H^+$) column; the eluent being 2N $NH_4OH$. The product was analysed by $^{13}C$ NMR analysis: trans/cis ratio 2:1.

### Example 8

A mixture of trans 2-cyano-6-chloro-3-azabicyclo[3.1.0]hexane (1.9 g), sodium cyanide (0.8 g) and isopropanol (12 ml) was stirred in a flask and heated under gentle reflux under a nitrogen atmosphere for 2.5 hours. After cooling the mixture was treated with diethyl ether (200 ml), filtered and the remaining liquid evaporated down.

The amount of product, recovered as a solid on standing, was 1.9 g. According to GLC analysis it contained 64% trans and 35% cis isomer.

The solid material was ground under hexane (80 ml) and isopropanol was added dropwise until the remaining insoluble material was filterable. Filtering yielded 0.6 g of a solid which, according to GLC analysis consisted of the trans isomer. From the mother liquid 1.3 g was recovered of a product which, according to GLC analysis, NMR and 1R spectrography comprised 50% of the cis isomer.

The trans isomer (0.6 g) was mixed with sodium cyanide (0.2 g) and isopropanol (3 ml) and heated under reflux for 1.5 hours. After cooling, the mixture was treated with diethylether and filtered. The remaining liquid was evaporated down. The amount of product recovered was 0.6 g containing 64% of the trans isomer.

Example 9

A mixture of trans 2-cyano-6-exochloro-3-azabicyclo[3.1.0]hexane (0.5 g), sodium cyanide (0.2 g) and isopropanol (3 ml) was stirred in a flask and heated under reflux for 1.5 hours. After allowing to cool the mixture was treated with diethylether, filtered and the remaining liquid evaporated down. A yellow liquid was obtained solidifying on standing, in an amount of 0.5 g. According to GLC analysis it contained 65% trans and 31% of the cis isomer.

**Claims**

1. A process for the isomerisation of a cis or trans 2-cyano-3-azabicyclo[3.1.0]hexane, characterized in that a compound of the general formula:

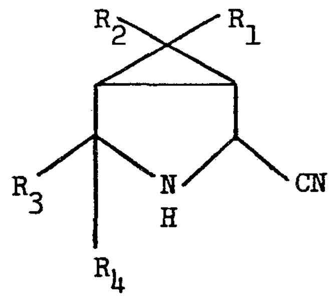

(I)

wherein the group CN and the

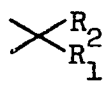

moiety are in a cis or trans configuration and $R_1$, $R_2$, $R_3$ and $R_4$ each independently represents a hydrogen or halogen atom or an alkyl group with up to 6 carbon atoms which may optically be substituted by up to 3 halogen atoms, is contacted with a solvent in the presence of a compound containing, or capable of generating, free cyanide moieties.

2. A process as claimed in claim 1, characterized in that a compound is isomerized in which each of $R_1$, $R_2$, $R_3$ and $R_4$ represents a hydrogen atom.

3. A process as claimed in either claim 1 or claim 2, characterized in that a cyanide salt, hydrogen cyanide, a cyanide-containing exchange resin or an aldehyde or ketone cyanohydrin, is present in the reaction mixture.

4. A process as claimed in claim 3, characterized in that an alkali metal cyanide is present in the reaction mixture.

5. A process as claimed in any one of claims 1 to 4, characterized in that as the solvent an alkanol having up to 6 carbon atoms, a ketone, an aromatic hydrocarbon, an ether, a sulphone or a sulphoxide is used.

6. A process as claimed in any one of claims 1 to 5, characterized in that as solvent isopropanol is used.

7. A process as claimed in any one of claims 1 to 6, characterized in that the isomerization is carried out at a temperature in the range of from ambient temperature to 200°C.

8. A process as claimed in any one of claims 1 to 7, characterized in that the isomerization is carried out at a temperature in the range of 80 to 100°C.

**Patentansprüche**

1. Verfahren zur Isomerisation eines cis- oder trans-2-Cyano-3-azabicyclo[3.1.0]hexans, dadurch gekennzeichnet, dass eine Verbindung der allgemeinen Formel:

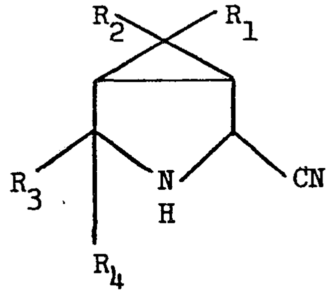

(I)

worin die Gruppe CN und der Rest

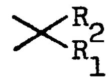

in einer cis-Konfiguration oder einer trans-Konfiguration vorliegen und $R_1$, $R_2$, $R_3$ und $R_4$ unabhängig voneinander jeweils ein Wasserstoffatom oder Halogenatom oder eine Alkylgruppe mit bis zu 6 Kohlenstoffatomen, die gewünschtenfalls durch bis zu 3 Halogenatome substituiert sein kann, bedeuten, mit einem Lösungsmittel in Gegenwart einer Verbindung in Berührung gebracht wird, die freie Cyanidreste enthält oder zur Bildung freier Cyanidreste befähigt ist.

2. Verfahren nach Anspruch 1, dadurch gekennzeichnet, dass eine Verbindung der Formel (I) isomerisiert wird, worin jeder der Reste $R_1$, $R_2$, $R_3$ und $R_4$ ein Wasserstoffatom bedeutet.

3. Verfahren nach Anspruch 1 oder 2, dadurch gekennzeichnet, dass in dem Reaktionsgemisch ein Cyanidsalz, Cyanwasserstoff, ein Cyanid enthaltendes Austauschharz oder ein Aldehyd- oder Ketoncyanhydrin enthalten ist.

4. Verfahren nach Anspruch 3, dadurch gekennzeichnet, dass in dem Reaktionsgemisch ein Alkalimetallcyanid enthalten ist.

5. Verfahren nach einem der Ansprüche 1 bis 4, dadurch gekennzeichnet, dass als Lösungsmittel ein Alkanol mit bis zu 6 Kohlenstoffatomen, ein Keton, ein aromatischer Kohlenwasserstoff, ein Äther, ein Sulfon oder ein Sulfoxid verwendet wird.

6. Verfahren nach einem der Ansprüche 1 bis 5, dadurch gekennzeichnet, dass als Lösungsmittel Isopropanol verwendet wird.

7. Verfahren nach einem der Ansprüche 1 bis 6, dadurch gekennzeichnet, dass die Isomerisation bei einer Temperatur in dem Bereich von etwa Umgebungstemperatur bis zu 200°C ausgeführt wird.

8. Verfahren nach einem der Ansprüche 1 bis 7, dadurch gekennzeichnet, dass die Isomerisation bei einer Temperatur in dem Bereich von 80 bis 100°C ausgeführt wird.

**Revendications**

1. Procédé pour l'isomérisation d'un cis ou trans 2-cyano-3-azabicyclo[3.1.0]hexane, caractérisé en ce qu'un composé de la formule générale:

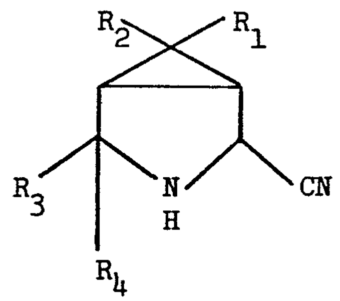

(I)

dans laquelle le groupe CN et la portion

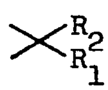

sont dans une configuration cis ou trans et $R_1$, $R_2$, $R_3$ et $R_4$ représentent chacun indépendamment un atome d'hydrogène ou d'un halogène ou un groupe alcoyle ayant jusqu'à 6 atomes de carbone qui peut éventuellement être substitué par jusqu'à 3 atomes d'halogène, est mis en contact avec un solvant en présence d'un composé contenant, ou capable de produire, des portions cyanure libres.

2. Procédé selon la revendication 1, caractérisé en ce qu'on isomérise un composé dans lequel $R_1$, $R_2$, $R_3$ et $R_4$ représentent chacun un atome d'hydrogène.

3. Procédé selon l'une des revendications 1 et 2, caractérisé en ce qu'un sel d'acide cyanhydrique, de l'acide cyanhydrique, une résine échangeuse contenant des groupes cyanure ou une aldéhyde ou cétone cyanhydrine est présent dans le mélange réactionnel.

4. Procédé selon la revendication 3, caractérisé en ce qu'un cyanure de métal alcalin est présent dans le mélange réactionnel.

5. Procédé selon l'une quelconque des revendications 1 à 4, caractérisé en ce que comme solvant on utilise un alcanol ayant jusqu'à 6 atomes de carbone, une cétone, un hydrocarbure aromatique, un éther, une sulfone ou un sulfoxyde.

6. Procédé selon l'une quelconque des revendications 1 à 5, caractérisé en ce que comme solvant on utilise l'isopropanol.

7. Procédé selon l'une quelconque des revendications 1 à 6, caractérisé en ce que l'isomérisation est effectuée à une température comprise entre la température ambiante et 200°C.

8. Procédé selon l'une quelconque des revendications 1 à 7, caractérisé en ce que l'isomérisation est effectuée à une température comprise entre 80 et 100°C.